## Europäisches Patentamt

## European Patent Office

## Office européen des brevets

(11) Veröffentlichungsnummer: **0 101 818**
**B1**

(12) **EUROPÄISCHE PATENTSCHRIFT**

(45) Veröffentlichungstag der Patentschrift:
**12.10.88**

(51) Int. Cl.⁴: **A 61 L 17/00**

(21) Anmeldenummer: **83106143.7**

(22) Anmeldetag: **23.06.83**

(54) **Chirurgisches Nahtmaterial aus einem Kunststoffmonofil.**

(30) Priorität: **30.07.82 DE 3228428**

(43) Veröffentlichungstag der Anmeldung:
**07.03.84 Patentblatt 84/10**

(45) Bekanntmachung des Hinweises auf die Patenterteilung:
**12.10.88 Patentblatt 88/41**

(84) Benannte Vertragsstaaten:
**AT BE CH DE FR GB IT LI LU NL SE**

(56) Entgegenhaltungen:
**DE-U-8 221 647**
**US-A-4 034 763**

**H. Saechtling, Kunststoff-Taschenbuch, 1979, Seiten 74-75**

(73) Patentinhaber: **MONOFIL- TECHNIK Gesellschaft für Synthese Monofile mbH, Industriegebiet Reutherstrasse Postfach 1328, D-5202 Hennef/Sieg (DE)**
Patentinhaber: **Krahmer, Gerhard M., Auf der Hedwigshöhe 4c, D-5064 Rösrath 3 (DE)**

(72) Erfinder: **Weber, Josef, Ringstrasse 63, D-5202 Hennef (DE)**
Erfinder: **Spielau, Paul, Dr., Van- Gogh- Platz 10, D-5210 Troisdorf- Eschmar (DE)**
Erfinder: **Fenske, Jürgen, Erich- Klausener- Strasse 10, D-5090 Leverkusen (DE)**
Erfinder: **Schrick, Hans- Jürgen, Rubensstrasse 129, D-5210 Troisdorf- Eschmar (DE)**
Erfinder: **Krahmer, Gerhard, Auf der Hedwigshöhe 4C, D-5064 Rösrath 3 (DE)**

(74) Vertreter: **Müller- Gerbes, Margot, Friedrich- Breuer- Strasse 112, D-5300 Bonn 3 (Beuel) (DE)**

# 0 101 818

**Beschreibung**

Die Erfindung betrifft ein chirurgisches Nahtmaterial aus einem Kunststoffmonofil.

Für chirurgische Nahtmittel werden eine Reihe unterschiedlicher Materialien eingesetzt, die den daraus hergestellten Nahtmitteln ihre charakteristischen Anwendungsmöglichkeiten verleihen. Bekannt sind einerseits die resorbierbaren Nahtmaterialien, wie Catgut, chromiert und plain sowie Polyglykolsäure und zum anderen die nicht resorbierbaren Nahtmaterialien aus Leinenzwirn, Baumwollzwirn, Seide, Polyamide, Polyester, Polyolefine, Tantal oder Stahl. Die Verwendung von Polytetrafluoräthylen als chirurgisches Nahtmaterial ist in der US-Patentschrift 4 034 763 beschreiben.

Die herausragenden Eigenschaften von z. B. Seide aus Nahtmaterial sind ihre hohe Reißkraft, Geschmeidigkeit und gute Knüpfbarkeit. Nachteile sind Dochtwirkung, die zu einer Abwehrreaktion des Körpergewebes als Folge führt und Abbau des Nahtmaterials im Gewebe und damit einhergehende Festigkeitsverminderung der Gewebenaht. Leinenzwirn hat ähnliche Nachteile und Vorteile wie Seide und konnte sich aber aufgrund des geringeren Preises sehr lange behaupten.

Eine Übersicht über die unterschiedlichen Nahtmittel und ihre Vor- und Nachteile sowie ihre Einsatzmöglichkeiten findet sich beispielsweise bei P. F. Nockemann "Die chirurgische Naht", Georg Thieme Verlag Stuttgart. New York 1980.

Chirurgische Nahtmaterialien aus Kunststoffen übertreffen Nahtmaterialien aus Naturfasern sowie Catgut in ihren Eigenschaften in vieler Hinsicht. Die am meisten herausragende Eigenschaft ist deren deutlich größere Reißkraft. In diesem Punkte werden Kunststoffäden nur noch von Metallfäden übertroffen. Dabei behalten Fäden aus Kunststoff im Gegensatz zu Fäden aus Naturstoffen ihre Festigkeit auch im Gewebe nahezu unverändert bei.

Beispielsweise liegt die Reißkraft von Polyamidfäden um 30 % über der von Seide oder Zwirn. Polyesterfäden weisen eine noch höhere Reißkraft auf.

Neben der Reißkraft, die von der Fadenstärke abhängt und die ein Maß für die Knotenfestigkeit des Nahtmittels ist, sind auch die Oberflächenbeschaffenheit, Elastizität und Quellbarkeit von Bedeutung für die Knotenzuverlässigkeit. Ferner müssen die Eigenschaften der Sterilität und der Gewebeverträglichkeit gewährleistet sein.

Bei der hohen Reißkraft von Kunststoffäden ist ihre gleichzeitige Geschmeidigkeit von besonderem Vorteil. Allerdings ist die Geschmeidigkeit mit hoher Elastizität gekoppelt. Eine hohe Elastizität, gepaart mit der hohen Reißkraft, bedingt bei den synthetischen monofilen Nahtmitteln einen wesentlichen Nachteil. Für den Operateur erweist sich die hohe Elastizität der Kunststoffäden beim Knoten als störend. Er kann nur schwer die richtige Nahtspannung abschätzen. Darüber hinaus besteht die Gefahr, daß die abgeschnittenen Enden des beim Anziehen gedehnten Fadens in den Knoten zurückschlüpfen und so der Knoten geöffnet wird. Diese geringe Knotensicherheit macht bei allen Kunststoffäden in mehr oder weniger starkem Maße eine spezielle Knotentechnik erforderlich. Wegen der elastischen Dehnbarkeit müssen die Fäden vor und während des Knotens besonders stark angezogen werden. Dies ist andererseits wegen der hohen Fadenfestigkeit noch mit Kräften möglich, die andere Fäden bereits zerreißen würden. Das bei der Naht gefaßte Gewebe gerät auf diese Weise aber leicht unter überhöhten Druck, so daß es geschädigt wird.

Zur Verbesserung der Handhabbarkeit und Herabsetzung der Dehnung von chirurgischen Nahtmitteln aus Kunststoff sind daher auch multifile Fäden entwickelt worden. Ein geflochtener oder multifiler Faden ist zwar geschmeidiger als ein monofiler Faden, hat dafür aber bei der Verwendung als chirurgische Nahtmittel eine gewisse Sägewirkung auf das Gewebe. Daher wird in vielen Fällen dieser nachteiliger Rauhigkeit multifiler Fäden durch Aufbringen eines zusätzlichen Glättemittels entgegengewirkt.

Der Erfindung liegt die Aufgabe zugrunde, ein monofiles chirurgisches Nahtmaterial aus Kunststoff zur Verfügung zu stellen, das eine geringe Dehnung und damit eine verbesserte Handhabbarkeit aufweist.

Diese Aufgabe wird mit der Erfindung gelöst. Sie besteht darin, bei einem chirurgischen Nahtmaterial ein verstrecktes Kunststoffmonofil aus Polyvinylidenfluorid (PVDF) zu verwenden.

Die monofilen Fäden aus PVDF gemäß der Erfindung besitzen eine hohe Reißkraft und Geschmeidigkeit, weisen jedoch gegenüber bekannten Kunststoffmonofilen eine niedrigere Elastizität und damit geringere Dehnung auf. Diese Eigenschaften-Kombination verbessert die Handhabbarkeit als chirurgisches Nahtmaterial. Ferner zeichnen sich solche monofilen Fäden aus PVDF z. B. gegenüber Materialien aus Polypropylen durch eine verringerte Neigung zum Aufspleißen aus.

Der erfindungsgemäße Faden ist somit besser in der Handhabbarkeit und im Knotensitz.

Zur Unterscheidung von anderen Materialien kann er mit einem geeigneten Farbstoff eingefärbt werden. Die Fadenstärken liegen vorzugsweise zwischen 0,07 und 0,5 mm. Das Material kann in üblicher Weise in seiner Festigkeit durch Einstellung des Kristallinitätsgrades, z. B. mittels der Kühlbedingungen nach dem Extrudieren des Monofilaments, optimiert werden. Knotenreißkraft und Reißdehnung eines geknoteten Fadens können als Funktion des Verstreckungsgrades ermittelt und entsprechend eingestellt werden.

Bevorzugt weist das erfindungsgemäße Monofil als Nahtmaterial eine axiale Verstreckung im Verhältnis von 1 : 3 bis 1 : 8 vorzugsweise 1 : 4 bis 1 : 6 auf. Wesentlich ist, daß die Reißkraft bzw. Knotenreißkraft, die natürlich von der Dicke des Monofils abhängt, den an das Nahtmaterial gestellten Anforderungen genügt, wobei diese um das zwei- bis dreifache eingehalten werden. Die Knoten-Reißdehnung beträgt bei den erfindungsgemäßen Monofilen nur 8 bis 20 % vorzugsweise 8 bis 18 %. Sie liegt damit im Durchschnitt um 50 % niedriger als die vergleichbaren Monofile für Nahtmaterial aus Polyolefinen oder Polyamid.

2

Darüber hinaus haben Testversuche ergeben, das PVDF-Monofile eine sehr gute Gewebeverträglichkeit aufweisen und keine Entzündungen hervorrufen.

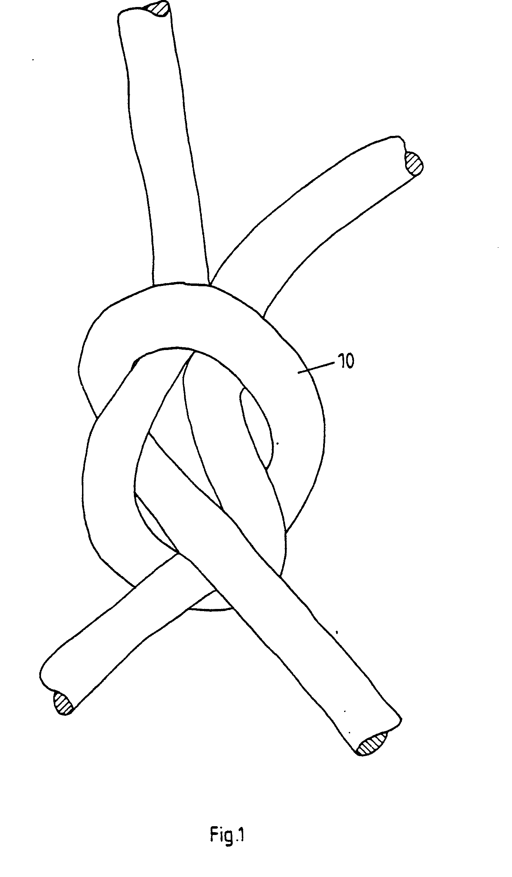

In der Zeichnung ist in Figur 1 ein PVDF-Monofil 10 in geknoteter Form gezeigt. Das PVDF-Monofil weist eine sehr hohe Reißfestigkeit, Knotenreißfestigkeit, Rutschfestigkeit, relative kleine Reißdehnung, sehr hohe Geschmeidigkeit, glatte Oberfläche, kein Spleißen und gute Durchmesserkonstanz auf.

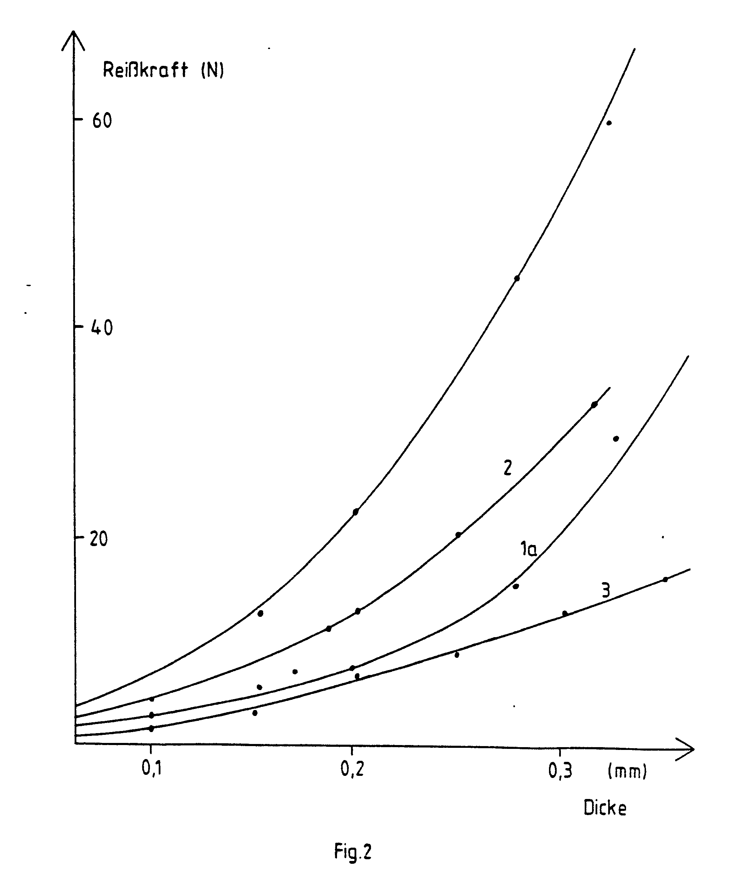

In Figur 2 ist ein Diagramm der Reißkraft in Abhängigkeit von der Dicke des Monofils dargestellt. Hierbei zeigt die Kurve 1 die Reißkraft eines PVDF-Monofils, Kurve 1a die Knotenreißkraft des PVDF-Monofils, Kurve 2 die Reißkraft eines PP-Monofils und Kurve 3 den Sollwert der Knotenreißkraft.

Die Werte der Knotenreißkraft, Reißkraft und Reißdehnung von PVDF-Monofilen gemäß der Erfindung und handelsüblichen Polypropylenmonofilen verschiedener Dicke wurden gemessen. Die Monofile werden nach Dicken sortiert bezeichnet, die tatsächlich vorhandene Dicke in mm wird gemessen, die Reißkraft und Knotenreißkraft in N gemessen, ggf. als Mittelwert aus mehreren Messungen, ebenso die Reißdehnung in %. Des weiteren wird noch der Sollwert der Knotenreißkraft für chirurgisches Nahtmaterial angegeben. Aus den nachfolgenden gemessenen Werten ist die deutliche Verbesserung in allen Werten bezüglich des Einsatzes als chirurgisches Nahtmaterial von PVDF gegenüber PP ersichtlich.

| | PVDF-Monofil | | | Polypropylen-Monofil | |
|---|---|---|---|---|---|

**Dicke (mm)**

| | | | | | |
|---|---|---|---|---|---|
| 1) | 0,14 | (1,0 metric) | | 0,7 metric, 6 - 0 | |
| 2) | 0,19 | (1,5 metric) | | 1,5 metric, 4 - 0 | |
| 3) | 0,27 | (2,5 metric) | | 2   metric, 3 - 0 | |
| 4) | 0,33 | (3,0 metric) | | 3   metric, 2 - 0 | |
| 5) | 0,38 | (3,5 metric) | | | |

**Dicke (Meßwert mm)**

| | | | | | |
|---|---|---|---|---|---|
| 1) | 0,152 | (1) | (0,145 - 0,160) | 0,100 (2) | (0,097 - 0,103) |
| 2) | 0,196 | (1) | (0,192 - 0,204) | 0,185 (2) | (0,175 - 0,195) |
| 3) | 0,279 | (1) | (0,265 - 0,295) | 0,25  (2) | (0,245 - 0,28) |
| 4) | 0,326 | (1) | (0,295 - 0,365) | 0,315 (2) | (0,3   - 0,33) |
| 5) | 0,370 | (1) | (0,345 - 0,415) | | |

**Knotenreißkraft Meßwert (N)**

| | | | | |
|---|---|---|---|---|
| 1) | 4,8 | (4,2 - 5,3) | - | |
| 2) | 7,1 | (7,3 - 9,0) | - | |
| 3) | 15,7 | (14,4 - 17,0) | - | |
| 4) | 24,9 | (21,6 - 26,5) | - | |
| 5) | 38,7 | (35   - 42) | - | |

**Knotenreißkraft Sollwert (N)**

| | | | | |
|---|---|---|---|---|
| 1) | 1,5 | 0,4 | 0,7 | 0,25 |
| 2) | 3,5 | 0,7 | 3,5 | 0,7 |
| 3) | 8,5 | 3,5 | 6,5 | 1,5 |
| 4) | 12,5 | 6,5 | 12,5 | 6,5 |
| 5) | 16 | 8,5 | | |

**Reißkraft (N)**

| | | | | |
|---|---|---|---|---|
| 1) | 12,5 | (12,0 - 13,1) | 4,3 | |
| 2) | 22,4 | (20,7 - 24,1) | 11,7 | |
| 3) | 45,6 | (33,2 - 39,2) | 20,5 | |
| 4) | 60 | (55,1 - 62,5) | 33 | |
| 5) | 81,5 | (77,5 - 86) | | |

**Reißdehnung (%)**

| | | | |
|---|---|---|---|
| 1) | 15(14 - 16) | 35 | |
| 2) | 15(14 - 16) | 40 | |
| 3) | 15(13 - 16) | 30 | |
| 4) | 16(15 - 17) | 35 | |
| 5) | 16(15 - 18) | | |

(1) = Mittelwerte und Streubereich von 5 Fäden (= 25 Messungen)
(2) = Mittelwerte und Streubereich von 1 Faden (= 4 Messungen)

## Patentansprüche

1. Chirurgisches Nahtmaterial aus einem verstreckten Monofil aus Kunststoff, dadurch gekennzeichnet, daß der Kunststoff Polyvinylidenfluorid ist.

2. Monofil nach Anspruch 1, dadurch gekennzeichnet, daß es im Verhältnis von 1 : 3 bis 1 : 6 verstreckt ist.

3. Monofil nach Anspruch 1 oder 2, dadurch gekennzeichnet, daß er eine Knoten-Reißdehnung von 8 bis 20 % vorzugsweise 8 bis 18 % aufweist.

## Claims

1. Surgical suture material formed of a stretched plastics monofilament, characterised in that the plastics is polyvinylidene fluoride.

2. Monofilament according to claim 1, characterised in that it is stretched in a ratio of 1 : 3 to 1 : 6.

3. Monofilament according to claim 1 or 2, characterised in that it possesses a knot extension on tearing of 8 to 20 %, preferably 8 to 18%.

## Revendications

1. Suture chirurgicale en un monofilament de matière synthétique étiré caractérisé par le fait que la matière synthétique est le poly(fluorure de vinylidène).

2. Monofilament selon la revendication 1, caractérisé par le fait qu'il est étiré dans un rapport de 1 : 3 à 1 : 6.

3. Monofilament selon les revendications 1 ou 2, caractérisé par le fait qu'il présente un allongement à la rupture des noeuds de 8 à 20 %, de préférence de 8 à 18 %.

Fig.1

Fig.2